# EUROPEAN PATENT SPECIFICATION

(11) **EP 1 317 502 B1**
(45) Date of publication and mention of the grant of the patent: **25.07.2007**
(21) Application number: 01964390.7
(22) Date of filing: 23.08.2001
(51) Int. Cl.: C08J 3/03, C08F 26/06, C08F 26/10, A61K 8/00

(54) **POLYMERIC COMPOSITION**
POLYMERZUSAMMENSETZUNG
COMPOSITION POLYMERE

(30) Priority: 15.09.2000 US 663010; 15.02.2001 US 784268; 15.06.2001 US 881906; 15.06.2001 US 882415; 15.06.2001 US 882418
(43) Date of publication of application: 11.06.2003
(73) Proprietor: ISP INVESTMENTS INC., Wilmington, Delaware 19801 (US)
(72) Inventor: HOOD, David, K., Basking Ridge, NJ 07920 (US); KOPOLOW, Stephen, L., Plainsboro, NJ 08536 (US); TALLON, Michael, Aberdeen, NJ 07747 (US); KWAK, Yoon, Tae, Woodcliff Lake, NJ 07677 (US); SENAK, Laurence, Livingston, NJ 07039 (US); PATEL, Drupesh, Lake Hiawatha, NJ 07034 (US); MC KITTRICK, John, Jersey City, NJ 07305 (US)
(74) Representative: Ford, Michael Frederick
(86) International application number: PCT/US2001/026417
(87) International publication number: WO 2002/022722

(56) References cited:
- US-A- 5 717 045
- US-A- 5 767 169
- US-A- 5 997 855
- US-A- 6 090 865

## Description

### BACKGROUND OF THE INVENTION

### 1. Field of the Invention

This invention relates to polymeric compositions, and, more particularly, to a non-continuous, vinyl lactam polymeric composition having two phases therein, particularly suitable for making clear to translucent, water-resistant, color inkjet receptive films on a substrate.

### 2. Description of the Prior Art

Polymeric compositions of vinyl lactam monomers generally are one-phase, soluble, high viscosity materials. These compositions are found in a variety of commercial applications such as film formers, dye transfer inhibitors, rheology modifiers, dispersants, excipients, and drug delivery. Aqueous gels of these monomers can also be prepared by light covalent or associative crosslinking of polymer chains resulting in a highly swellable, one phase material of high viscosity. These compositions are effective thickeners for use in personal care formulations such as hair care products.

Niessner, in U.S. Pats. 5,149,750 and 5,180,804, disclosed finely divided, water-swellable gel-like, water-swellable copolymers by polymerization of comonomers in the presence of a surfactant.

Liu, in U.S. Pat. 5,997,855, described a homogeneous terpolymer for hair care use, however, without a crosslinking agent.

Kopolow, in U.S. Pat. 5,130,121, described personal care compositions containing a stabilized cosmetically-active product obtained by in situ polymerization of a water-soluble vinyl monomer in the presence of discrete microdroplets of a cosmetically-active oil in water.

Blankenburg, in U.S. Pats. 5,635,169 and 6,107,397, also described uncrosslinked aqueous copolymer dispersions of nonionic water-soluble monomers with N-vinyl groups, and hydrophobic monomers.

Steckler, in U.S. Pat. 3,878,175, disclosed highly absorbent spongy gel polymer materials by simultaneously copolymerizing and partially crosslinking a comonomer mixture of an alkyl acrylate and a heterocyclic N-vinyl monomer containing a carbonyl functionality in the presence of a hydrophobic liquid diluent in which the final polymer is insoluble.

Markus, in U.S. Pat. 2,810,716, described a process for making swellable resins by copolymerizing monomers in the presence of a water-soluble non-redox divalent-ion containing salt.

Tseng, in U.S. Pats. 5,393,854 and 5,717,045 disclosed a one-phase, aqueous gel of crosslinked copolymers of vinyl pyrrolidone and dimethylaminoethyl methacrylate for use in hair care products. The crosslinking agent was 1-vinyl-3-(E)-ethylidene pyrrolidone. The gels had a Brookfield viscosity of between 60,000 and 100,000.

These references illustrate the desire of the art to produce a continuous network of polymer molecules, or microgel which is a one-phase system, and of high viscosity.

Another application for polymeric compositions is in color ink-jet printing. The advent of color inkjet printing has been instrumental in fueling the print-on-demand revolution and has also created a number of challenges. Often, the surface of the desired media does not possess the necessary properties for accepting the ink-jet ink. This results in long dry times and/or a poor ink-jet image. It has long been recognized that a surface treatment or media coating plays a critical role in the final print quality. Numerous media coatings are known in the art. They may contain any number of components and often consist of more than one layer. These ink-receptive coatings generally contain at least one hydrophilic polymer; often poly(vinylpyrrolidone) (PVP). In contrast to the teaching of the thickener art for personal care products, networked, highly swellable polymeric systems are undesirable in this application. Soluble PVP brings many benefits to properly formulated media coatings including rapid ink dry time, excellent print quality, highly resolved circular dots, and high, uniform optical density. Furthermore, copolymers of vinylpyrrolidone (VP) along with other suitable comonomers, such as dimethylaminoethyl methacrylate, acrylic acid, or vinyl acetate, have been used separately or in conjunction with PVP, to further optimize performance. Unfortunately, their resistance to water penetration can be weak. It is desired to provide long-term, excellent water-resistant qualities for such films.

Accordingly, it is an object of the present invention to provide an aqueous polymeric composition which is not a gel but a combination of film forming polymer and substantially uniformly dispersed minute resinous particles that under suitable light magnification, shows the presence of two discrete phases therein, one of which is a water soluble polymer and the other is in situ-formed, water-insoluble resinous particles.

Accordingly, another object of the invention is to provide an advantageously water-resistant color inkjet-receptive film coated with the defined polymer composition of the invention, which is capable of being printed from a color ink-jet printer to form superior water-resistant color images thereon.

Accordingly, another object of the invention is to provide a water-resistant film with improved light fastness, UV protection, and bleed reduction.

Accordingly, another object of the invention is to demonstrate the utility of these compositions for a variety of applications, including, but not limited to, dye transfer inhibitors, rheology modifiers, refractive index modifiers, UV protectants, fragrance and silicone delivery, dispersants, excipients, drug delivery, and in personal care formulations.

Another object of the invention is to provide an aqueous polymeric system for delivering active materials ordinarily present in personal care formulations, such as silicones, fragrances, sunscreens, and the like; in nutrient, medicament and pharmaceutical formulations, such as drugs, for example, aspirin, and syrups and the like.

Another object herein is to provide a suitable process for making such a polymeric composition.

Still another object of the present invention to provide a post-treatment composition and process for two-phase, aqueous polymeric compositions which can provide advantageous water-resistant polymeric coatings or films, or optionally, a highly-swellable polymeric gel.

A specific object of the invention is to provide a post-polymerization of such two-phase composition by further polymerization with additional crosslinker, to provide the desired water-resistant color inkjet-receptive film coated with the defined post-treated polymer composition of the invention, which film is capable of being printed from a color ink-jet printer to form superior water-resistant color images thereon.

Another specific object of the invention is to provide a post-treatment of such two-phase composition by further blending with an additional proteinaceous compound, to provide the desired water-resistant color inkjet-receptive film coated with the defined post-treated polymer composition of the invention, which film is capable of being printed from a color ink-jet printer to form superior water-resistant color images thereon.

Among the other objects of this invention to provide a new and improved rheology modifier composition to adjust the rheological properties of commercial products.

A feature of the invention is the provision of an aqueous polymeric composition suitable for forming clear to translucent, water-resistant coatings on a substrate.

### IN THE DRAWINGS

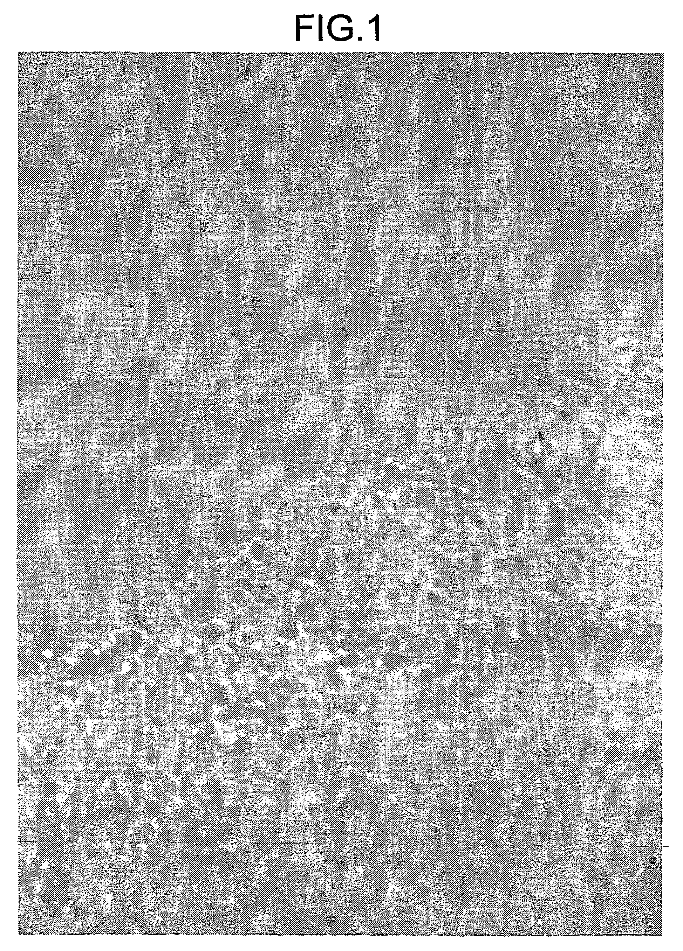

FIGURE 1 is a photomicrograph of the aqueous polymeric composition of the invention showing the presence of two discrete phases therein.

FIGURE 2 is a graphical representation of Brookfield viscosity of the invention composition vs. Φ the volume fraction of particles in the composition.

### SUMMARY OF THE INVENTION

What is described herein is a stable, aqueous polymeric composition which forms a clear to translucent film upon application to a substrate comprising, by weight, 5-75% of (a) a water-soluble polymer having (b) in situ-formed, substantially water-insoluble resinous particles of said polymer substantially uniformly dispersed therein, and (c) 25-95% of water.

Preferably the polymer is polyvinylpyrrolidone (PVP), poly(vinylcaprolactam) (PVCL), a copolymer of PVP and/or PVCL, and, optionally, one or more comonomers, including comonomers such as dimethylaminopropyl(meth)acrylamide (DMAPMA) and dimethylaminoethyl(meth)acrylate (DMAEMA). Preferably the polymer is a vinyl lactam polymer, optionally copolymerized with a methacrylate/acrylate and/or methacrylamide/acrylamide comonomer.

In this invention the composition includes particles having a size of < 500 µ, preferably < 100 µ, and optimally between > 1 nm and < 500 µ.

Suitably the composition includes a substantially water-insoluble polymer which is a crosslinked or branched polymer, neutralized and/or quaternized, and/or functionalized quaternized. The ratio of (a):(b) is 20-95% to 5-80%, preferably 20-75% to 25-80%, and the crosslinking agent is a substantially water-insoluble compound, preferably pentaerythritol triallyl ether (PETE), or pentaerythritol tetraacrylate (PETA), preferably at least partially soluble in water, and the crosslinking agent is present in an amount of 0.02-0.5% by weight of said composition, most preferably 0.05-0.3%.

In this invention the composition has a Brookfield viscosity of 1-45 Pa·s (1,000 to 45,000 cps), preferably 2-20 Pa·s (2,000 to 20,000 cps).

As a feature of the invention there is provided herein a process for making a stable, aqueous polymeric composition which includes the steps of providing a reaction mixture of a water-soluble vinyl monomer, optionally with one or more water-soluble comonomers, a predetermined amount of a crosslinking agent and water, heating the mixture, then periodically adding a predetermined amount of an initiator, and polymerizing at about 30-130°C, optionally further including the step of diluting with water during or after the polymerization.

Suitably the crosslinking agent is present in an amount of 0.02-0.5 wt.% based on monomers present, and preferably is PETE or PETA, and the initiator is an azo initiator.

Another feature of the invention is the provision of formulations containing the above-described composition, made by such process, and films of the composition on a substrate.

The compositions herein may be dried if desired to provide the polymeric composition as a solid, and, if desired, the water soluble polymer extracted with a solvent. The dried stable polymeric composition thereby includes, by weight, (a) 20% to 95% of a water-soluble polymer, and (b) 5% to 80% of in situ-formed, substantially water-insoluble resinous particles of said polymer substantially uniformly dispersed therein.

Another feature of the invention is the provision of a system for delivering an active material which includes (1) a stable, aqueous polymeric composition of, by weight, (a) a water-soluble polymer having (b) in situ-formed, substantially water-insoluble resinous particles of said polymer substantially uniformly dispersed therein, and (c) 25-95% of water; and (2) an active material dispersed in said composition.

The active material for delivery from the polymeric system herein preferably is a hydrophobic, substantially water-insoluble material, which ordinarily is present in personal care formulations, such as silicones and fragrances, or an active material present in nutrient, medicament and pharmaceutical formulations, such as aspirin, syrups, and the like.

Still another feature is a post-treatment product and process for making a water-resistant polymeric coating or a strongly-swellable polymeric gel which comprises (1) providing a stable, aqueous two-phase polymeric composition by the steps of (a) a reaction mixture in a reaction vessel of a water-soluble vinyl monomer, optionally with one or more water-soluble comonomers, a predetermined amount of a first crosslinking agent, and water, heating the mixture, then periodically adding a predetermined amount of an initiator, and polymerizing at about 30-130°C, optionally removing said resultant two-phase composition from the reaction vessel, adding a predetermined amount of additional crosslinker or proteinaceous compound thereto, optionally casting the mixture onto a support, and optionally heating to further polymerize or blend the mixture.

The compositions herein are effective rheology modifiers for use in aqueous and alcoholic formulations.

### DETAILED DESCRIPTION OF THE INVENTION

In accordance with this invention, there is provided herein an aqueous polymeric composition having two phases therein, a water-soluble polymeric phase and a discrete, water-insoluble polymer particle phase which is generated in-situ during the polymerization of the monomers. In the preferred forms of the invention, the polymerization is carried out in aqueous solution of a vinyl lactam monomer, such as vinyl pyrrolidone or vinyl caprolactam. Optionally a comonomer may be present to form a copolymer. Suitable comonomers include methacrylate/acrylate monomers, such as dimethylaminoethyl(meth)acrylate (DMAEMA) and/or methacrylamide/acrylamide monomers, such as dimethylaminopropylacrylamide (DMAPMA).

This stable, aqueous polymeric composition forms a clear to translucent film upon application to a substrate and comprises, by weight, 5-75% of (a) a water-soluble polymer having (b) in situ-formed, substantially water-insoluble resinous particles of said polymer substantially uniformly dispersed therein, and (c) 25-95% of water.

The active material for delivery herein usually are those actives ordinarily found in personal care products such as skin and hair care products, or drugs which are administered in a sustained or time release mode. Both water-insoluble and water-soluble actives can be used. Generally the active material is dispersed in the polymeric composition by neutralization or chelation.

The post-treatment step of the invention includes removing the composition from the reaction vessel, optionally drying, adding additional crosslinking agent or proteinaceous compound, and further polymerizing or blending to form the desired water-resistant coating. Usually the reaction mixture is applied to a substrate, such as a polyester film, before polymerizing or processing. A highly-swellable polymeric gel is made by limiting the amount of additional crosslinker added.

The invention will now be illustrated in more detail by reference to the following examples.

### PREPARATION OF TWO-PHASE, AQUEOUS POLYMERIC COMPOSITIONS

### EXAMPLE 1

### Two-Phase Polymeric Composition of VP/DMAPMA/Neutralized

1. To a 2-I kettle fitted with a nitrogen inlet tube, thermocouple, agitator, and feed lines was added 87.15 g of vinyl pyrrolidone monomer, (VP), 697 g DI water and 0.275 g (0.25% based upon monomer) of pentaerythritol triallyl ether (PETE) as crosslinker.
2. Purged with nitrogen subsurface for 30 minutes.
3. Heated to 70°C.
4. In a separate container weighed out 22.69 g of dimethylaminopropyl methacrylamide (DMAPMA).
5. With kettle temperature at 70°C, stop subsurface nitrogen purge and purged above surface. Precharged 1.1 g DMAPMA from container.
6. Started continuous addition of the remaining DMAPMA (21.86 g) over 210 minutes at a flow rate 0.11 ml/minute. Once the DMAPMA flow started, initiated with first shot of Vazo® 67 in isopropanol (IPA) (Time 0).
7. Initiator was added in 5 separate shots at 0, 30, 60, 150 and 210 minutes. 0.2 g of Vazo® 67 in 1.0 g IPA was added for each shot and two 0.5 g IPA washes were made.
8. Held the reaction temperature overnight at 70°C.
9. When residual VP level was below 400 ppm, diluted the batch with 266.7 g of DI water.
10. Cooled batch to 50°C.
11. Neutralized the batch with conc. HCl to pH of 6.2-6.8 at 50°C. Room temperature pH was 6.8-7.2. Required approximately 14 g of conc. HCl.
12. Added 0.15 to 0.19% BTC 50 NF as preservative.
13. A two-phase, aqueous polymeric composition as shown in the Figure was obtained having the properties shown in Table 1 below.

### EXAMPLE 2

The process of Example 1 was repeated using 5 separate shots of 0.3 g each of Vazo® 67 in 1.0 g of IPA. A similar polymeric composition as in Example 1 was obtained having the properties shown in Table 1.

### EXAMPLE 3

The process of Example 1 was repeated using 5 separate shots of 0.4 g each of Vazo® 67 in 1 g of IPA, and 0.3 g of crosslinker. A similar polymeric composition was obtained, having the properties shown in Table 1.

### Test Methods

Draw-downs from a 10% aqueous solution of the polymer composition of Examples 1-3 were cast onto a polyester substrate using a #38 Mayer bar and allowed to dry in an oven at 100°C to give a dry coating thickness of approximately 9 microns.

Coated samples were then printed using a HP 832C printer at 600 DPI in "HP Premium Photo Paper" mode. Individual blocks of cyan(C), magenta(M), yellow(Y), and black(K), approximately 2.54 cm x 4.38 cm (1" X 1.75") in size, were printed side by side. Small blocks of C, M, Y, and K, approximately are printed repeatedly down one edge of the page to provide a built-in time-line for measuring offset time as described below.

Off-set time is the minimum time required for no ink to transfer to a cover sheet placed on top of the print when contacted with a 1.81 Kg (4-lb) roller immediately after printing. Ink transfer is determined at the point where the OD after testing dropped by a value of 0.2 units. Fast offset times are most desirable.

Water-resistance was measured by the following standard test procedure.

Water resistance was tested by placing the printed sheet at a 45° angle and dripping 10 ml of water at a constant rate (2 ml/min) over the surface for a maximum of 5 minutes. The samples were then judged by following rating system:

| | |
|---|---|
| Poor - | All ink removed in less than 1 minute. |
| Fair - | Most or all ink removed between 1 and 5 minutes. |
| Moderate - | Some (<50%) loss of ink after 5 minutes. |
| Good - | Very slight (<10%) loss of ink with minimal running. |
| Very Good - | 100% water resistance with no change in appearance. |

### Results

The results of these tests, shown in Table 1, establish that the 2-phase polymeric composition of the invention exhibits an advantageous water-resistant property as well as desired viscosity and haze properties, and low offset times.

**TABLE 1**

| Ex. No. | X-Linker (%) | Initiator (g/shot) | Residual VP (ppm) | Haze (NTU) | Visco Pa.s ((cps)) | Waterproof (min) | Offset Time* (Min) |
|---|---|---|---|---|---|---|---|
| 1 | 0.25 | 0.2 | 139 | | 28.2 (28,200) | 10 | < 1 |
| 2 | 0.25 | 0.3 | 151 | 25.2 | 13 (13,000) | 11 | < 1 |
| 3 | 0.3 | 0.4 | 173 | 45.7 | 12.8 (12,800) | 7 | < 1 |

| | | | | | | | |
|---|---|---|---|---|---|---|---|
| * The offset times of Examples 1-3 were < 1 minute. | | | | | | | |

### EXAMPLE 4

### Polymeric Composition of PVP

1. To a 2-I kettle fitted with a nitrogen inlet tube, thermocouple, agitator, and feed lines was added 131.81 g of VP, 756 g Dl water and 0.197 g PETE (0.15% based upon monomer).
2. Purged with nitrogen subsurface for 30 minutes.
3. Heated to 70°C.
4. Initiator was added at 0 and 30 minutes. 0.48 g of Vazo® 67 in 1.5 g IPA was added for each shot and two 1.0 g IPA washes were made.
5. Held the reaction temperature overnight at 70°C.
6. When residual VP was below 400 ppm, diluted the batch with 320.04 g DI water.
7. Cooled batch to 50°C.
8. Added 0.15 to 0.19% BTC 50 NF as preservative.
9. The product was a 2-phase, polymerization composition with 40 to 70% resinous particles, whose soluble fraction had a molecular weight of 1,200,000 to 1,500,000.

### EXAMPLE 5

### Polymeric Composition of VP/DMAPMA/Quaternized with Diethyl Sulfate

1. To a 2-I, kettle fitted with a nitrogen inlet tube, thermocouple, agitator, and feed lines was added 96.00 g of VP, 702.7 g DI water and 0.36 g PETE (0.30% based upon monomer).
2. Purged with nitrogen subsurface for 30 minutes.
3. Heated to 70°C.
4. In a separate container weighed out 24.0 g DMAPMA and 74.7 g DI water. Purged with nitrogen.
5. When kettle temperature was at 70°C, stopped subsurface nitrogen purge and purged above surface. Precharged 4.94 g DMAPMA/water from container.
6. Started continuous addition of the remaining DMAPMA/water (93.76 g) over 210 minutes. Flow rate 0.48 ml/minute. Once DMAPMA/water flow started, initiated with first shot of Vazo® 67 in IPA (Time 0).
7. Initiator was added at 0, 30, 60, 150 and 210 minutes. 0.44 g of Vazo 67 in 1.3 g IPA was added for each shot and two 0.7 g IPA washes were made.
8. Held the reaction temperature overnight at 70°C.
9. When residual VP was below 400 ppm, diluted the batch with 297.5 g DI water.
10. Cooled batch to 50°C.
11. Neutralized the batch with 19.56 g diethyl sulfate (DES) over 60 minutes; at flow rate of 0.28 g/ml.
12. Stirred for 2 hours.
13. Product.

### EXAMPLE 6

### VP/DMAPMA/PETE Neutralized with Benzophenone-4

1. To a 2-I kettle fitted with a nitrogen inlet tube, thermocouple, agitator, and feed lines was added 87.15 g of HPVP, 630 g DI water and 0.33 g PETE (0.30% based upon monomer).
2. Purged with nitrogen subsurface for 30 minutes.
3. Heated to 70°C.
4. Weighed out 22.69 g DMAPMA and 67 g DI water. Purged with nitrogen.
5. When kettle temperature was at 70°C, stopped subsurface nitrogen purge and purged above surface. Precharged 4.23 g DMAPMA/water from container.
6. Started a continuous addition of the remaining DMAPMA/water (85.46 g) over 210 minutes. Flow rate 0.40 ml/minute. Once DMAPMA/water flow started, initiated with first shot of Vazo® 67 in IPA (Time 0).
7. Initiator was added at 0, 30, 60, 150 and 210 minutes. 0.4 g of Vazo 67 in 1.0 g IPA was added for each shot and two 0.5 g IPA washes were made.
8. Held the reaction temperature overnight at 70°C.
9. When residual VP was below 400 ppm, diluted the batch with 266.7 g DI water.
10. Cooled batch to 50°C.
11. Neutralized the batch with benzophenone-4, 5 to 99 mole % (2 to 38.6 g respectively). Continued neutralization with sulfuric acid to pH of 6.8 to 7.8 at 50°C.
12. Cooled and discharged.
13. Product.

### EXAMPLE 7

### VP/DMAPMA/PETA

1. To a 2-1 kettle fitted with a nitrogen inlet tube, thermocouple, agitator, and feed lines was added 104.58 g of HPVP, 756 g DI water and 0.59 g pentaerythritol tetra acrylate (0.30% based upon monomer).
2. Purged with nitrogen subsurface for 30 minutes.
3. Heated to 70°C.
4. In a separate container, weighed out 27.23 g DMAPMA and 80.4 g Dl water. Purged with nitrogen.
5. When kettle temperature was at 70°C, stopped subsurface nitrogen purge and purged above surface. Precharged 5.38 g DMAPMA/water from container.
6. Started continuous addition of the remaining DMAPMA/water (102.25 g) over 210 minutes. Flow rate 0.52 ml/minute. Once DMAPMA/water flow started, initiated with first shot of Vazo®67 in IPA (Time 0).
7. Initiator was added at 0, 30, 60, 150 and 210 minutes. 0.16 g of Vazo® 67 in 1.0 g IPA was added for each shot and two 0.5 g IA washes were made.
8. Held the reaction temperature overnight at 70°C.
9. When VP was below 400 ppm, diluted the batch with 266.7 g DI water.
10. Cooled batch to 50°C.
11. Neutralized the batch with conc. sulfuric acid to pH of 6.6 to 7.8 at 25°C.
12. Added 0.15 to 0.19% BTC 50 NF as preservative.
13. Product.

### EXAMPLE 8

### Crosslinked Vinyl Caprolactam/DMAPMA Copolymer

1. To a 2-I kettle fitted with a nitrogen inlet tube, thermocouple, agitator and feed lines was added 130.7 g vinyl caprolactam, 128.7 g DI water, 171.6 g ethanol, and 0.88 g PETE (0.6% based upon monomer).
2. Purged with nitrogen for 30 minutes.
3. Heated to 70°C.
4. In a syringe pump was added 32.98 g DMAPMA and 179.6 g DI water.
5. At 70°C added 40 ml of the DMAPMA/water mixture to the kettle and added the first shot of initiator, 0.075 g Vazo® 67 in 0.75 g ethanol. Washed with 0.75 g ethanol.
6. Started addition of the remaining DMAPMA/water mixture (Time 0) from the syringe pump at a rate of 0.34 ml/min, added over 480 minutes.
7. At time 60, 120, 180, 240, 300, 360, 420 and 480 minutes added a shot of Vazo® 67, 0.075 g in 0.75 g ethanol. Washed with 0.75 g ethanol.
8. Held at 70°C overnight.
9. Cooled reaction to 30°C and added 415.6 g Dl water.
10. Mixed until uniform and then added 544.4 g Dl water and 15.38 g hydrochloric acid.
11. Mixed for 2 hours. Adjusted pH to 6.6 to 7.8 with hydrochloric acid, if necessary.
12. Added 0.15 to 0.19% BTC-50 NF as preservative.
13. Product.

### EXAMPLE 9

### VP/DMAEMA/PETE Process

1. To a 2-I kettle fitted with a nitrogen inlet tube, thermocouple, agitator, and feed lines is added 87.15 g of HPVP, 630 g DI water and 0.33 g (0.30% based upon monomer) pentaerythritol triallyl ether.
2. Purged with nitrogen subsurface for 30 minutes.
3. Heated to 70°C.
4. In a separate container, weighed out 22.69 g DMAEMA and 67 g DI water. Purged with nitrogen.
5. When kettle temperature was at 70°C, stopped subsurface nitrogen purge and purged above surface. Precharged 4.23 g DMAEMA/water from container.
6. Started continuous addition of the remaining DMAEMA/water (85.46 g) over 210 minutes. Flow rate 0.40 ml/minute. Once DMAEMA/water flow started initiator addition with first shot of Vazo 67 in IPA (Time 0).
7. Initiator was added at 0, 30, 60, 150, and 210 minutes. 0.4 g of Vazo 67 in 1.0 g IPA was added for each shot and two 0.5 g IPA washes were made.
8. Held the reaction temperature overnight at 70°C.
9. When VP was below 400 ppm, diluted the batch with 266.7 g DI water.
10. Cooled batch to 50°C.
11. Neutralized the batch with conc. HCl to pH of 6.2 to 6.8 at 50°C. Room temperature pH will be 6.8 to 7.2. Required approximately 14 g of conc. HCl.
12. Added 0.15 to 0.19% BTC 50 NF as preservative.
original 24 hour observations. The particle size on the methanol dispersed material was measured using a Microtrak UPA and found to be about 4 Tm.

### COMPARATIVE EXAMPLE 12

An aqueous solution of 119.64 g of vinyl pyrrolidone monomer, 0.36 g pentaerythritol triallyl ether (PETE), 0.6 g of Vazo 67, and 480 g water was charged to a kettle and purged with nitrogen. The reaction mixture was then heated to 65°C while stirring at 650 rpm. Within 25 minutes the product became so viscous that the reaction was stopped. The product was a continuous gel only.

### COMPARATIVE EXAMPLE 13

An aqueous solution of 119.64 g of vinyl pyrrolidone monomer, 0.36 g pentaerythritol triallyl ether (PETE), 0.23 g of Vazo 67, and 480 g water was charged to a kettle and purged with nitrogen. The reaction mixture was then heated to 65°C while stirring at 650 rpm. After 2 hours at 65°C, the reaction was heated to 95°C for 1 hour. The product was a viscous solution only.

### EXAMPLE 14

### DPI Film Coating Formulation

| Ingredient | Parts by Weight |
|---|---|
| VP/DMAPMA/PETE (Ex. 1) | 2.00 |
| PV-OH (88% hydrolyzed) | 8.00 |
| Sequrez® 755 (glyoxyl) | 0.75 |
| Water | 89.25 |
| | 100.00 |

### EXAMPLE 95

### UV Coating Formulation

| Ingredient | Parts by Weight |
|---|---|
| VP/DMAPMA/PETE/BENZO-4 (Ex. 6) | 2.00 |
| PV-OH (88% hydrolyzed) | 8.00 |
| Sequrez® 755 (glyoxyl) | 0.75 |
| Water | 89.25 |
| | 100.00 |

### EXAMPLE 16

### Sunscreen Cream

| Ingredients | | Wt.% |
|---|---|---|
| **PHASE A** | | |
| | Deionized water | 15.69 |
| | Disodium EDTA | 0.10 |
| | Acrylates/Steareth-20 Methacrylate Copolymer | 1.00 |
| | Acrylates Copolymer | 1.00 |
| | Hexylene Glycol | 1.00 |
| | Glyceryl Polymethacrylate and Propylene | |
| | Glycol and PVM/MA Copolymer | 0.50 |
| | VP/DMAPMA/PETE/Benzophenone-4 | |
| | Copolymer (Ex. 6) | 50.00 |
| | | |

| **PHASE B** | | |
|---|---|---|
| | Glyceryl Stearate and Behenyl Alcohol and Palmitic and Stearic Acid and Lecithin and Lauryl and Myristyl Alcohol and Cetyl Alcohol | 5.00 |
| | Oxybenzone | 3.00 |
| | Octyl Salicylate | 3.00 |
| | Tridecyl Neopentanoate | 2.00 |
| | Octyl Palmitate | 6.00 |
| | Myristyl Myristate | 1.00 |
| | | |

| **PHASE C** | | |
|---|---|---|
| | Deionized Water | 5.00 |
| | NaOH, 10% Solution | 1.26 |
| | | |

| **PHASE D** | | |
|---|---|---|
| | Diazolidinyl Urea and lodopropynyl Butylcarbamate | 0.50 |
| | Methyl Paraben | 0.20 |
| | Hexylene Glycol | 1.00 |
| | | |

| **PHASE E** | | |
|---|---|---|
| | Fragrance | 0.25 |

### PROCEDURE

1. Combine ingredients in Phase A and heat to 70-75°C.
2. Combine ingredients in Phase B and mix and heat to 70-75°C.
3. Add Phase B to Phase A under homogenization.
4. Add Phase C to the batch under homogenization and homogenize for 15 minutes.
5. Switch to propeller mixing and cool to 45°C.
6. Add Phase D at 45°C. Add Phase E at 40°C. QS with water.

The UV absorbance of the cream was enhanced by the presence of the polymeric composition of the invention therein, as compared to similar formulations without this composition, generally an increase of about 2-3 SPF numbers.

### EXAMPLE 17

### Clear Styling/Conditioning Gel

| Ingredients | Wt. % |
|---|---|
| Deionized Water | 74.60 |
| Ethanol (190 Proof) | 5.00 |
| VP/DMAPMA/PETE Copolymer (Ex. 1) | 20.00 |
| Dimethicone Copolyol | 0.10 |
| Caprylyl Pyrrolidone | 0.10 |
| Panthenol | 0.10 |
| 2,4-Dihydroxy-N-(3-hydroxypropyl)-3,3-Dimethyl Butanamide | |
| Diazolidinyl Urea and lodopropynyl Butylcarbamate | 0.10 |
| Perfume | qs |

### Manufacturing Procedure

1. In a vessel, add ethanol to water and stir until homogeneous.
2. Next, add VP/DMAPMA/PETE copolymer to the mixture and stir well until homogeneous.
3. Add dimethicone copolyol, panthenol and caprylyl pyrrolidone to the mixture and stir well after each addition until homogeneous.
4. Next, add diazolidinyl urea and iodopropynyl and butylcarbamate and stir well until homogeneous.

### EXAMPLE 18

### Rinse-Off Protection Hair Conditioner

| Ingredients | Wt. % |
|---|---|
| Deionized Water | 81.73 |
| Emulsifying Wax NF | 4.00 |
| Cetearyl Alcohol and Ceteareth-20 | 2.00 |
| Propylene Glycol | 1.00 |
| VP/DMAPMA/PETE Neutralized with Benzophenone-4 (Ex. 6) | 10.00 |
| Glycerin 99.7% | 0.50 |
| Lauryl Pyrrolidone | 0.25 |
| Citric Acid FCC, USP, Anhydrous | 0.02 |
| Propylene Glycol and Diazolidinyl Urea and Iodopropynyl butylcarbamate | 0.50 |

### Manufacturing Procedure

1. Heat the water, propylene glycol, glycerin, and citric acid to 80-85°C using continuous addition with a propeller stir rod.
2. Add the VP/DMAPMA/PETE neutralized with Benzophenone-4 and stir to homogeneous.
3. Combine in a separate vessel lauryl pyrrolidone, emulsifying wax NF, cetearyl alcohol and ceteareth-20, heating to 80-85°C mixing until homogeneous.
4. Add, product step 3, to the water phase with good agitation. Mix with continuous agitation for 10-20 minutes or longer. Maintain temperature at 80-85°C during this step.
5. Begin cooling with continuous agitation until approximately 45°C. Do not force cool.
6. Switch to a paddle mixing rod. Continue slow agitation and cool until a temperature of 30-35°C is reached. At 30-35°C add the propylene glycol and diazolidinyl urea and iodopropynyl butylcarbamate and continue mixing until 25°C is reached.

### EXAMPLE 19

### Polymeric Coatings on a Substrate

Films were cast of 0.5 mil thickness on a 8.26 x 10.16 x 0.16 cm (3.25 x 4 x 1/16 inch) glass plate from 11 % by weight aqueous polymeric solutions prepared (a) with no particles and (b) with the particle-containing polymeric solutions of Example 1 (51% particles). The resulting films which contained no UV protectant additives and had a coating volume of about 1.3 TL/cm². The UV (and SPF) spectroscopic results via a Solar Protection Measurement System indicated that the presence of polymeric particles in the film coating increased its ability to absorb UV radiation, and showed an increase in the SPF number from 2.3 to 5.3. The coating also was non-irritating, non-toxic, water-resistant and visually clear.

### EXAMPLE 20

### Pharmaceutical Tablet Composition

| | | |
|---|---|---|
| Acetaminophen | | 93.5% |
| PVP/PETE (in place of Polyplasdone K-90) | | 4% |
| Polyplasdone XL | | 2% |
| Magnesium Stearate | | 0.5% |
| | Total | 100% |

### EXAMPLE 21

### Perfume

0.25 g of D-limonene was mixed 0.25 g of the two-phase composition of poly(VP/DMAPMA/PETE)-HCl salt, and 2 ml H₂O water added, and mixed vigorously with a magnetic stirrer for 30 minutes. Then added 97.5 g of water was added, and mixed for 2 minutes. The emulsion was then centrifuged for 20 minutes at 3000 rpm to remove any free D-limonene. Free D-limonene was decanted off and the dispersed limonene-containing composition was a clear translucent solution. A sample of 0.25% D-limonene in water alone was also centrifuged and decanted as a control. D-limonene is an orange smelling fragrance which is insoluble in water. Indeed, the poly(VP/DMAPMA/PETE) containing formulation had a pungent orange smelling fragrance which was significantly stronger than the control. Furthermore, it demonstrates that nano-particle technology can be manipulated for perfumes, fragrance extenders like air-fresheners, as well as many other time released/smart delivery systems. GC analysis will confirm the level of D-limonene present.

### EXAMPLE 22

### Silicone Oil Delivery

A mixture of 0.5 g silicone 200 oil and 0.5 g poly(VP/DMAPMA/PETE)-HCl salt, (100% solids) was vigorously agitated by magnetic stirrer for 30 minutes. The polymer particles swelled in the oil as the oil entered the polymer composition. Then 5 ml of water was added and the mixture was stirred for an additional hour. A thick, white emulsion was obtained. Added 98.5 g of water to obtain a 0.5% silicone encapsulate with 0.5% polymer in water. Centrifuged the mixture for 2 hours at 3000 rpm and decanted to remove the non-encapsulated material, i.e. free silicone oil. The product was then analyzed by FT-IR for the presence of silicone, using a ZnSe disk, and drying to form a film. The solutions were optically clear. The IR profile for the product vs. silicone oil itself showed the presence of encapsulated silicone oil in the polymer composition and no free silicone.

### EXAMPLE 23

### Pharmaceutical Active Delivery System

Naproxen free acid, whose optical activity is [α]_{D}²⁵=+66°, 1.02 g was added to 40.77 g of VP/DMAPMA/PETE (10.9% solids, pH = 10.1). The final pH was 8.0. The final solution exhibited translucent properties.

### EXAMPLE 24

### Pharmaceutical Active Dispersion

Dried VP/DMAPMA/PETE/sulfuric acid 0.40 g was added to 0.05 g of Naproxen free acid. 14.85 g of ethanol was added to this mixture and then agitated for 2 hours. The solution was dried in a 60°C vacuum oven, in vacuo. 14.5 g of water was added to the dried mixture. Visually, the sample appeared uniformly dispersed. After 10 days some solid settling was observed, but the solution still appeared cloudy.

### POST-TREATMENT OF POLYMERIC COMPOSITIONS

### EXAMPLE 25

### Post-Treatment of (VP/DMAPMA/Neutralized/HCl)

Polymeric compositions of VP/DMAPMA/PETE neutralized with HCl, as described in Examples 1-3, were removed from the reactor vessel, and additional crosslinker was added. The crosslinker was a polyfunctional aziridine compound, Xama^{®} 7 from EIT, which was added at various weight percents of crosslinker, as given in the Table below. Draw-downs from a 10% aqueous solution of the mixture then were cast onto a polyester substrate using a #38 Mayer bar, allowed to dry and polymerize in an oven at 100°C for 10 minutes. A dry coating having a thickness of ~9 micron was obtained.

The thus-coated films were then printed using a HP 832C printer at 600 DPI in "HP Premium Photo Paper" mode. Individual blocks of cyan(C), magenta(M), yellow(Y), and black(K), approximately 2.54 x 4.45 cm (1" x 1.75") in size, were printed side by side.

The water-resistance of the film was measured by the following standard test procedure. The printed sheet was placed at a 45° angle and 10 ml of water at a constant rate of 2 ml/min was dripped over the surface. The samples were then judged by following rating system:

| | |
|---|---|
| Poor - | All ink removed in less than 1 minute. |
| Fair - | Most or all ink removed between 1 and 5 minutes. |
| Moderate - | Some (<50%) loss of ink after 5 minutes. |
| Good - | Very slight (<10%) loss of ink with minimal running. |
| Very Good - | 100% water resistance with no change in appearance after 1 hour. |

### Results

The results of these tests, shown in Table 2, establish that the polymeric composition post-treated with a suitable amount of additional crosslinking agent exhibited an advantageous water-resistant property.

**TABLE 2**

| Ex. No. | Additional Crosslinker Wt. (%) | Water Resistance (Time) of Film |
|---|---|---|
| 1 | 0.0 | 12 min 22 sec |
| 2 | 0.5 | 16 min 8 sec |
| 3 | 1.0 | > 1 hr |
| 4 | 1.5 | > 1 hr |

### EXAMPLE 26

100 g of VP/DMAPA/PETE·H₂SO₄, (10% solids) was mixed with 0.01 g of a red food dye and 2 g D-limonene (fragrance) to give a homogeneous dispersion. Addition of 0.5 g of Xama-7 (aziridine) crosslinker crosslinked the mixture. A hydrogel formed in 2 hours at RT without further mixing. The product was a homogeneous dispersion, red gel with an orange odor, similar to an air-freshener.

Similarly modified hydrogels can be formed with other commercial hydrogel systems such as contact lens membranes and hydrogel delivery systems.

### EXAMPLE 27

### Post Treatment/(VP/DMAPMA/PETE/Sulfuric Acid)/Test Methods

To improve water resistance to the transparent film, a blended polymeric composition comprised of VP/DMAPMA/PETE neutralized with sulfuric acid and a proteinaceous compound, such as skin gelatin, was prepared at 50/50 wt.%. This was accomplished by first mixing the gelatin into pre-heated 60°C water (10 wt. %). Second, VP/DMAPMA/PETE was mixed into the gelatin/water mixture. Draw-downs of this composition were cast onto a polyester substrate using a #38 Mayer bar and allowed to dry in an oven at 90°C for approximately 5 minutes to give a dry coating thickness of ~9 micron.

After standing 3 hours at room temperature, the coated sample was printed on using an Epson Stylus 800. Individual blocks of cyan(C), magenta(M), yellow(Y), and black(K), approximately 2.54 x 4.45 cm (1" x 1.75") in size, were printed side by side.

Water-resistance was measured by the following standard test procedure. Water resistance was tested by placing the printed sheet at a 45°C angle and dripping at least 10 ml of water at a constant rate (2 ml/min) over the surface. The samples were then judged by following rating system:

| | |
|---|---|
| Poor - | All ink removed in less than 1 minute. |
| Fair - | Most or all ink removed between 1 and 5 minutes. |
| Moderate - | Some (<50%) loss of ink after 5 minutes. |
| Good - | Very slight (<10%) loss of ink with minimal running. |
| Very Good - | 100% water resistance with no change in appearance. |

### Results

The results of these tests, shown in Table 3, establish that the two-phase polymeric composition, post treated with a proteinaceous compound, exhibits an advantageous water-resistant property. Note that for the VP/DMAPMA/PETE/Gelatin blend, no damage to imprinted surface was observed.

**TABLE 3**

| Sample | Gelatin (%) | Water Resistance Time |
|---|---|---|
| VP/DMAPMA/PETE | 0.0 | ~12 min 20 sec |
| VP/DMAPMA/PETE | 50 | >30 min |

### EXAMPLE 28

The results of the tests on Eamples 1-3 above, shown in Table 4 below establish that the 2-phase polymeric composition of the invention exhibits advantageous viscosity properties.

**TABLE 4**

| Ex. No. | Crosslinker (%) | Initiator (g/shot) | Viscosity Pa.s ((cps)) |
|---|---|---|---|
| 1 | 0.25 | 0.2 | 28.2 (28,200) |
| 2 | 0.25 | 0.3 | 13 (13,000) |
| 3 | 0.3 | 0.4 | 12.8 (12,800) |

### EXAMPLE 29

### Rheology Modifiers

A 5% aqueous polymer solution of VP/DMAPMA/PETE/sulfuric acid (Ex. 1) was thoroughly mixed with a 5% aqueous polymer solution of polyvinyl alcohol (PVOH). The Brookfield viscosity of each solution, and mixtures thereof, was determined to demonstrate the effect of rheology modification by the composition of the invention. Under visual inspection, the solutions appeared to be homogeneous. The results are presented in Table 5 below.

**TABLE 5**

| Test Solution | Brookfield | Viscosity Pa.s ((cps)) | Percent Scale |
|---|---|---|---|
| 5% VP/DMAPMA/PETE/Sulfuric Acid in Water (A) | LV,62,10RPM | 1.47 (1470) | 48.9 |
| 5% PVOH in water (B) | LV, 00, 30 RPM | 0.0052 (5.2) | 26.0 |
| 50/50 (w/w) mixture of A and B | LV, 62, 20 RPM | 0.464 (464) | 27.6 |

### EXAMPLE 30

A 5% aqueous polymer solution of VP/DMAPMA/PETE/sulfuric acid (Ex. 1) was thoroughly mixed with a 5% aqueous polymer solution of poly-2-ethyl-2-oxazoline (PEO). The Brookfield viscosity of each solution, and mixtures thereof, was performed to demonstrate the effect of rheology modification. Under visual inspection, the solution appeared to be homogeneous. The results are presented in Table 6 below.

**TABLE 6**

| Test Solution | Brookfield | Viscosity Pa·s ((cps)) | Percent Scale |
|---|---|---|---|
| 5% VP/DMAPMA/PETE/Sulfuric Acid in Water | LV, 62, 10 RPM | 1.47 (1470) | 48.9 |
| 5% PEO in water | LV, 00, 30 RPM | 0.004 (4.0) | 21.3 |
| 50/50 (w/w) mixture | LV, 62, 20 RPM | 0.339 (339) | 22.6 |

### EXAMPLE 31

A 1% aqueous polymer solution of VP/DMAPMA/PETE/sulfuric acid (Ex. 1) was thoroughly mixed with a 1% aqueous polymer solution of Kelcoloid HVF Algin (HVF), an alginate. The Brookfield viscosity of each solution and their combination was performed to demonstrate the effect of rheology modification. Under visual inspection, the solution exhibited turbidity. The results are presented in Table 7 below.

**TABLE 7**

| Test Solution | Brookfield | Viscosity Pa.s ((cps)) | Percent Scale |
|---|---|---|---|
| 1% VP/DMAPMA/PETE/Sulfuric Acid in Water | LV, 00, 6 RPM | 0.06 (60) | 58.8 |
| 1% HVF in water | LV, 62, 30 RPM | 0.709 (709) | 71.4 |
| 50/50 (w/w) mixture | LV, 61, 30 RPM | 0.02 (20) | 10.4 |

### EXAMPLE 32

A 1% aqueous polymer solution of PVP/PETE (Ex. 4) was thoroughly mixed with a 1% aqueous polymer solution of Kelcoloid HVF Algin (HVF). The Brookfield viscosity of each solution and their combination was performed to demonstrate the effect of rheology modification. Under visual inspection, the solution appeared to be homogeneous. The results are presented in Table 8 below.

**TABLE 8**

| Test Solution | Brookfield | Viscosity Pa·s ((cps)) | Percent Scale |
|---|---|---|---|
| 1 % PVP/PETE in Water | LV, 00, 12 RPM | 0.0124 (12.4) | 24.8 |
| 1% HVF in water | LV, 62, 30 RPM | 0.709 (709) | 71.4 |
| 50/50 (w/w) mixture | LV, 61, 12 RPM | 0.1295 (129.5) | 26.1 |

The volume fraction, Φ₁ of particles in the composition of the invention is determined by the following procedure.
(1) The two-phase aqueous polymeric composition is prepared as in the examples above.
(2) The known amount of the composition is passed through a resin bed to remove insoluble particles.
(3) A water soluble solution remains.
(4) The solution is subjected to light to determine its differential Refractive Index.
(5) The amount of soluble polymer which passed through the resin bed is determined.
(6) Φ = 1;(#5/#2).

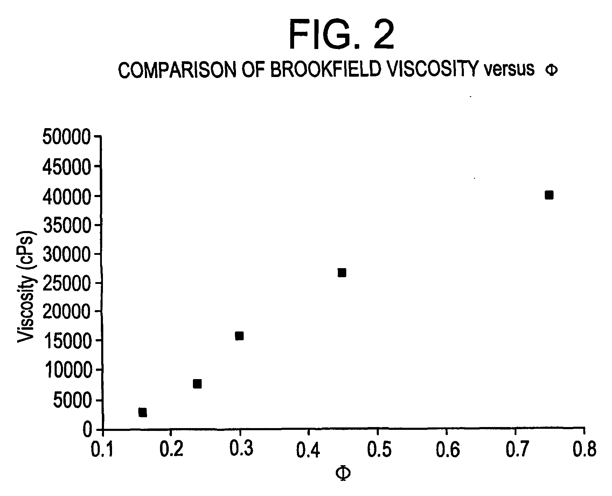

Figure 2 shows a plot of Brookfield viscosity vs. Φ, the volume fraction of particles in the 2-phase polymeric composition of the invention. The graph shows a dramatic increase in viscosity of the solution with an increase in the volume fraction of the particles therein, indicating it is an effective rheology modifier.

The compositions of the invention may be admixed, if desired, with one or more of the following commercially available rheology modifiers:
Acrylic polymers, crosslinked acrylic polymers, alginates, associative thickeners, carrageenan, microcrystalline cellulose, carboxymethylcellulose sodium, hydroxyethylcellulose, hydroxypropylcellulose, hydroxypropylmethylcellulose, methylcellulose, guar and guar derivatives, locust bea gum, organoclays, polyethylene, polyethylene oxide, polyvinylpyrrolidone, silica, water-swellable clay, xanthan gum and pigments (inorganic).

Product applications of the rheology modifier of the invention include the following:
Coatings, cementitious compounds, contrast mediums, wrinkle masking, cryoprotectants, detergents, marking Instruments, flocculation moderators, personal care formulations such as skin/hair care, including shampoo, conditioner, gels and creams, pharmaceutical, such as bioadhesives, syrups and excipients), lubricating oil additives, lubricants, adhesives and cosmetics.

### EXAMPLE 33

### Refractive Index Modification

VP/DMAPMA/PETE copolymer was neutralized with 4-benzophenone (Escalol® 557). The refractive index of the film was measured using a Model 2010 Prism Coupler on the bulk material by pressing the film against a prism. The result of this test demonstrates the ability to modify the refractive index of these polymeric film compositions by strategically selecting the neutralization acid.

**TABLE 9**

| **Sample** | **Refractive Index** |
|---|---|
| VP/DMAPMA/Sulfuric Acid | 1.52 |
| VP/DMAPMA/PETE/HCl | 1.52 |
| VP/DMAPMA/PETE/4-Benzophenone | 1.55 |

### EXAMPLE 34

### Other Commercial Uses for the Invention Aqueous Polymers Containing In Situ-Formed Polymeric Particles

1. UV protectants (coatings)
2. Sunscreen
3. Drug delivery systems (smart-delivery, smart-release)
4. Transdermal drug systems
5. Sizing (fabric coating)
6. Dye transfer inhibition
7. Autowaxes
8. Agricultural coatings/delivery
9. Personal care (hair care applications)
10. Abrasives (industrial and personal)
11. Encapsulates systems (entrapments)
12. Dispersants
13. Electro/optical systems

## Claims

1. A stable, aqueous polymeric composition which forms a clear to translucent film upon application to a substrate comprising, by weight, 5-75% of (a) a water-soluble polymer having (b) in situ-formed, water-insoluble resinous particles of said polymer substantially uniformly dispersed therein, wherein said water-insoluble resinous particles are a cross-linked or branched polymer, and (c) 25-95% of water.

2. A composition according to claim 1 wherein said polymer is polyvinylpyrrolidone (PVP), poly(vinylcaprolactam) (PVCL), a copolymer of PVP and/or PVCL, and, optionally, one or more comonomers, including dimethylaminopropyl(meth)acrylamide (DMAPMA) and/or dimethylaminoethyl(meth)acrylate (DMAEMA).

3. A composition according to claim 1 wherein said particles are <500µ.

4. A composition according to claim 3 wherein said particles are >1nm and <500µ.

5. A composition according to claim 1 wherein said substantially water-insoluble polymer is optionally neutralized and/or functionally neutralized and/or quaternized, and/or functionalized quaternized.

6. A composition according to claim 1 wherein the ratio of (a):(b) is 20-95% to 5-80%, preferably 20-75% to 25-80%.

7. A composition according to claim 5 wherein said crosslinking agent is a substantially water-insoluble compound, preferably pentaerythritol trialllyl ether (PETE) or pentaerythritol tetraacrylate (PETA).

8. A composition according to claim 5 wherein said functional neutralisation acid is a UV active, preferably based upon derivatives of cinnamic and/or benzoic and/or sulfonic and/or acetic and/or terephthalic and/or maleic acids, or a pharmaceutically active acid, or silicone, optionally wherein said functional neutralisation acid modifies the refractive index of the polymer film composition.

9. A composition according to claim 7 wherein said crosslinking agent is present in an amount of 0.02-0.5% by weight of said composition.

10. A composition of claim 1 having a Brookfield viscosity of 1-45 Pa.s (1,000 to 45,000 cps).

11. A composition of claim 1 wherein said polymer is a vinyl lactam polymer, optionally copolymerised with a methacrylate/acrylate and/or methacrylamide/acrylamide comonomer.

12. A composition of claim 1 which includes an active material suspended in said composition which is a personal care, nutrient or a pharmaceutically active material.

13. A dry, stable polymeric composition for delivering an active material comprising by weight, (a) 20% to 95% of a water-soluble polymer, (b) 5% to 80% of in situ-formed, water-insoluble resinous particles of said polymer substantially uniformly dispersed therein and (c) a water-insoluble active material suspended in said composition.

14. A process for making the stable, aqueous polymeric composition of claim 1, which comprises providing a reaction mixture of a water-soluble vinyl monomer, optionally with one or more water-soluble comonomers, a predetermined amount of a water-insoluble crosslinking agent and water, heating the mixture, then periodically adding a predetermined amount of an initiator, and polymerising at 30-130°C, optionally including the step of diluting with water and/or alcohol during or after the polymerisation, preferably in which said crosslinking agent is present in an amount of 0.02-0.5 wt.% based on monomers present.

15. A formulation containing the composition of claim 1.

16. A film of the composition of claim 1 on a substrate.

17. A composition according to claim 1 which is dried to provide the polymeric composition as a solid.

18. A dry, stable polymeric composition comprising, by weight, (a) 20% to 95% of a water-soluble polymer and (b) 5% to 80% of in situ-formed, water-insoluble resinous particles of said polymer substantially uniformly dispersed therein.

19. A post-treatment process for making a water-resistant polymeric coating or a strongly-swellable polymeric gel which comprises providing a stable, aqueous two-phase polymeric composition by the steps of forming a reaction mixture in a reaction vessel of a water-soluble vinyl monomer, optionally with one or more water-soluble comonomers, a predetermined amount of a first water-insoluble crosslinking agent, and water, heating the mixture, then periodically adding a predetermined amount of an initiator, and polymerising at 30-130ºC, optionally removing said resultant two-phase composition from the reaction vessel, adding a predetermined amount of additional crosslinker or proteinaceous compound thereto, optionally coating the mixture onto a support, and optionally heating to further polymerise the mixture.

## Patentansprüche

1. Stabile wässrige Polymerzusammensetzung, die beim Auftragen auf ein Substrat einen klaren bis durchsichtigen Film bildet, umfassend 5-75 Gew.-% (a) eines wasserlöslichen Polymers mit (b) in-situ-gebildeten, wasserunlöslichen Harzteilchen des Polymers, die im Wesentlichen einheitlich darin dispergiert sind, wobei es sich bei den wasserunlöslichen Harzteilchen um ein vernetztes oder verzweigtes Polymer handelt, und (c) 25-95 Gew.-% Wasser.

2. Zusammensetzung nach Anspruch 1, worin es sich bei dem Polymer um Polyvinylpyrrolidon (PVP), Poly(vinylcaprolactam) (PVCL), ein Copolymer aus PVP und/oder PVCL und fakultativ einem oder mehreren Comonomeren, umfassend Dimethylaminopropyl(meth)acrylamid (DMAPMA) und/oder Dimethylaminoethyl(meth)-acrylat (DMAEMA), handelt.

3. Zusammensetzung nach Anspruch 1, worin die Teilchen < 500 µm sind.

4. Zusammensetzung nach Anspruch 3, worin die Teilchen > 1 nm und < 500 µm sind.

5. Zusammensetzung nach Anspruch 1, worin das im Wesentlichen wasserunlösliche Polymer fakultativ neutralisiert und/oder funktionell neutralisiert und/oder quaternisiert und/oder funktionell quaternisiert ist.

6. Zusammensetzung nach Anspruch 1, worin das Verhältnis (a):(b) 20-95 % zu 5-80 %, vorzugsweise 20-75 % zu 25-80 %, beträgt.

7. Zusammensetzung nach Anspruch 5, worin das Vernetzungsmittel eine im Wesentlichen wasserunlösliche Verbindung ist, vorzugsweise Pentaerythrittriallylether (PETE) oder Pentaerythrittetraacrylat (PETA).

8. Zusammensetzung nach Anspruch 5, worin die Säure zur funktionellen Neutralisierung eine UV-aktive Säure, vorzugsweise auf Basis von Derivaten von Zimt- und/oder Benzoe- und/oder Sulfon- und/oder Essig- und/oder Terephthal- und/oder Maleinsäure, oder eine pharmazeutisch aktive Säure oder Silicon ist, wobei die Säure zur funktionellen Neutralisierung gegebenenfalls den Brechungsindex der Polymerfilmzusammensetzung verändert.

9. Zusammensetzung nach Anspruch 7, wobei das Vernetzungsmittel in einer Menge von 0,02-0,5 Gew.-% der Zusammensetzung vorhanden ist.

10. Zusammensetzung nach Anspruch 1 mit einer Brookfield-Viskosität von 1-45 Pa·s (1.000 bis 45.000 cps).

11. Zusammensetzung nach Anspruch 1, wobei das Polymer ein, gegebenenfalls mit einem Methacrylat/Acrylat- und/oder Methacrylamid/Acrylamid-Monomer copolymerisiertes, Vinyllactampolymer ist.

12. Zusammensetzung nach Anspruch 1, die ein aktives Material umfasst, das in der Zusammensetzung, bei der es sich um Körperpflege, einen Nährstoff oder ein pharmazeutisch aktives Material handelt, suspendiert ist.

13. Trockene stabile Polymerzusammensetzung für die Zufuhr eines aktiven Materials, umfassend (a) 20 bis 95 Gew.-% eines wasserlöslichen Polymers, (b) 5 bis 80 Gew.-% in-situ-gebildeter, wasserunlöslicher Harzteilchen des Polymers, die im Wesentlichen einheitlich darin dispergiert sind, und (c) ein wasserunlösliches aktives Material, das in der Zusammensetzung dispergiert ist.

14. Verfahren zur Herstellung der stabilen, wässrigen Polymerzusammensetzung nach Anspruch 1, das Folgendes umfasst: Bereitstellen eines Reaktionsgemischs aus einem wasserlöslichen Vinylmonomer, gegebenenfalls mit einem oder mehreren wasserlöslichen Comonomeren, einer vorbestimmten Menge eines wasserunlöslichen Vernetzungsmittels und Wasser, Erhitzen des Gemischs, wonach periodisch eine vorbestimmte Menge eines Initiators zugesetzt wird, sowie Polymerisieren bei 30-130 °C, wobei gegebenenfalls ein Schritt des Verdünnens mit Wasser und/oder Alkohol während des Polymerisierens durchgeführt werden kann, wobei das Vernetzungsmittel vorzugsweise in einer Menge von 0,02 bis 0,5 Gew.-% bezogen auf die vorhandenen Monomere vorhanden ist.

15. Formulierung, umfassend die Zusammensetzung nach Anspruch 1.

16. Film aus der Zusammensetzung nach Anspruch 1 auf einem Substrat.

17. Zusammensetzung nach Anspruch 1, die getrocknet wird, um die Polymerzusammensetzung als Feststoff bereitzustellen.

18. Trockene stabile Polymerzusammensetzung, umfassend (a) 20 bis 95 Gew.-% eines wasserlöslichen Polymers und (b) 5 bis 80 Gew.-% in-situ-gebildeter, wasserunlöslicher Harzteilchen des Polymers, die im Wesentlichen einheitlich darin dispergiert sind.

19. Nachbehandlungsverfahren zur Herstellung einer wasserbeständigen Polymerbeschichtung oder eines stark quellfähigen Polymergels, Folgendes umfassend: Bereitstellen einer stabilen wässrigen Zwei-Phasen-Polymerzusammensetzung durch die Schritte des Herstellens eines Reaktionsgemischs in einem Reaktionsgefäß aus einem wasserlöslichen Vinylmonomer, gegebenenfalls mit einem oder mehreren wasserlöslichen Comonomeren, einer vorbestimmten Menge eines ersten wasserunlöslichen Vernetzungsmittels und Wasser, Erhitzen des Gemischs, worauf das periodische Zusetzen einer vorbestimmten Menge eines Initiators folgt, und Polymerisieren bei 30-130 °C, fakultative Entfernung der resultierenden Zwei-PhasenZusammensetzung aus dem Reaktionsgefäß, Zusetzen einer vorbestimmten Menge eines zusätzlichen Vernetzungsmittels oder einer proteinhältigen Verbindung, fakultative Beschichtung des Gemischs auf einen Träger und fakultatives Erhitzen für das weitere Polymerisieren des Gemischs.

## Revendications

1. Composition polymère aqueuse stable qui forme un film clair à translucide lors d'une application à un substrat comprenant, en poids, 5 à 75 % de (a) un polymère soluble dans l'eau ayant (b) des particules résineuses insolubles dans l'eau formées in situ dudit polymère dispersées sensiblement uniformément à l'intérieur, dans laquelle lesdites particules résineuses insolubles dans l'eau sont un polymère réticulé ou ramifié, et (c) 25 à 95 % d'eau.

2. Composition selon la revendication 1, dans laquelle ledit polymère est la poly(vinylpyrrolidone) (PVP), le poly(vinylcaprolactame) (PVCL), un copolymère de PVP et/ou PVCL, et, facultativement, d'un ou plusieurs comonomères, comprenant le diméthylaminaopropyl (méth)acrylamide (DMAPMA) et/ou le (méth)acrylate de diméthylaminoéthyle (DMAEMA).

3. Composition selon la revendication 1, dans laquelle lesdites particules sont < 500 µ.

4. Composition selon la revendication 3, dans laquelle lesdites particules sont > 1 nm et < 500 µ.

5. Composition selon la revendication 1, dans laquelle ledit polymère sensiblement insoluble dans l'eau est facultativement neutralisé et/ou neutralisé d'un point de vue fonctionnel et/ou quaternisé, et/ou quaternisé fonctionnalisé.

6. Composition selon la revendication 1, dans laquelle le rapport de (a): (b) est de 20 à 95 % à 5 à 80 %, de préférence de 20 à 75 % à 25 à 80 %.

7. Composition selon la revendication 5, dans laquelle ledit agent de réticulation est un composé sensiblement insoluble dans l'eau, de préférence le pentaérythritol triallyl éther (PETE) ou le tétraacrylate de pentaérythritol (PETA).

8. Composition selon la revendication 5, dans laquelle ledit acide de neutralisation fonctionnel est actif sous UV, de préférence à base de dérivés d'acides cinnamique et/ou benzoïque et/ou sulfonique et/ou acétique et/ou téréphtalique et/ou maléique, ou un acide pharmaceutiquement actif, ou la silicone, facultativement dans laquelle ledit acide de neutralisation fonctionnel modifie l'indice de réfraction de la composition de film polymère.

9. Composition selon la revendication 7, dans laquelle ledit agent de réticulation est présent en une quantité de 0,02 à 0,5 % en poids de ladite composition.

10. Composition selon la revendication 1, ayant une viscosité de Brookfield de 1 à 45 Pa.s (1 000 à 45 000 cps).

11. Composition selon la revendication 1, dans laquelle ledit polymère est un polymère de vinyl lactame, facultativement copolymérisé avec un comonomère méthacrylate/acrylate et/ou méthacrylamide/acrylamide.

12. Composition selon la revendication 1, qui comprend une matière active mise en suspension dans ladite composition qui est un soin personnel, un nutriment ou une matière pharmaceutiquement active.

13. Composition polymère stable sèche destinée à délivrer une matière active comprenant en poids, (a) de 20 % à 95 % d'un polymère soluble dans l'eau, (b) de 5 % à 80 % de particules résineuses insolubles dans l'eau formées in situ dudit polymère dispersé sensiblement uniformément à l'intérieur et (c) une matière active insoluble dans l'eau mise en suspension dans ladite composition.

14. Procédé de fabrication de la composition polymère aqueuse stable de la revendication 1, qui comprend les étapes consistant à former un mélange de réaction d'un monomère de vinyl soluble dans l'eau, facultativement avec un ou plusieurs comonomères solubles dans l'eau, une quantité prédéterminée d'un agent de réticulation insoluble dans l'eau et de l'eau, chauffer le mélange, puis ajouter périodiquement une quantité prédéterminée d'un initiateur, et polymériser à 30 à 130°C, comprenant facultativement l'étape consistant à diluer avec de l'eau et/ou un alcool pendant ou après la polymérisation, de préférence dans lequel ledit agent de réticulation est présent en une quantité de 0,02 à 0,5 % en poids sur la base des monomères présents.

15. Formulation contenant la composition selon la revendication 1.

16. Film constitué de la composition selon la revendication 1 sur un substrat.

17. Composition selon la revendication 1, qui est séchée pour former la composition polymère sous forme d'un solide.

18. Composition polymère stable sèche comprenant, en poids, (a) de 20 % à 95 % d'un polymère soluble dans l'eau et (b) de 5 % à 80 % de particules résineuses insolubles dans l'eau formées in situ dudit polymère dispersées sensiblement uniformément à l'intérieur.

19. Procédé de post-traitement pour fabriquer un revêtement polymère résistant à l'eau ou un gel polymère ayant la faculté de gonfler fortement, qui comprend les étapes consistant à former une composition polymère à deux phases aqueuse stable par les étapes consistant à former un mélange de réaction dans une cuve de réaction d'un monomère de vinyle soluble dans l'eau, facultativement avec un ou plusieurs comonomères solubles dans l'eau, une quantité prédéterminée d'un premier agent de réticulation insoluble dans l'eau, et de l'eau, chauffer le mélange, puis ajouter périodiquement une quantité prédéterminée d'un initiateur, et polymériser à 30 à 130 °C, retirer facultativement ladite composition à deux phases résultante de la cuve de réaction, lui ajouter une quantité prédéterminée d'un agent de réticulation additionnel ou composé protéique, revêtir facultativement le mélange sur un support, et chauffer facultativement pour polymériser davantage le mélange.
